(19) Europäisches Patentamt
European Patent Office
Office européen des brevets

(11) EP 4 729 607 A1

(12) EUROPEAN PATENT APPLICATION
published in accordance with Art. 153(4) EPC

(43) Date of publication:
22.04.2026 Bulletin 2026/17

(21) Application number: 24823465.0

(22) Date of filing: 14.06.2024

(51) International Patent Classification (IPC):
C12N 5/02 (2006.01)    C12M 1/00 (2006.01)
C12M 3/00 (2006.01)    C12N 5/071 (2010.01)

(52) Cooperative Patent Classification (CPC):
C12M 1/00; C12M 3/00; C12N 5/0006; C12N 5/06

(86) International application number:
PCT/JP2024/021655

(87) International publication number:
WO 2024/257852 (19.12.2024 Gazette 2024/51)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 15.06.2023 JP 2023098735
23.06.2023 JP 2023103140

(71) Applicant: UBE Corporation
Ube-shi, Yamaguchi 755-8633 (JP)

(72) Inventors:
• OHYA, Shusei
Ichihara-shi, Chiba 290-0045 (JP)

• NISHIDA, Hiroshi
Ichihara-shi, Chiba 290-0045 (JP)
• MATSUBAYASHI, Akihiro
Ichihara-shi, Chiba 290-0045 (JP)
• GUO, Lei
Ichihara-shi, Chiba 290-0045 (JP)
• KAMADA, Asumi
Ichihara-shi, Chiba 290-0045 (JP)

(74) Representative: Plougmann Vingtoft a/s
Strandvejen 70
2900 Hellerup (DK)

Remarks:
The complete document including Reference
Table(s) and the Sequence Listing(s) can be
downloaded from the EPO website

(54) METHOD FOR CRYOPRESERVING CELLS, METHOD FOR CULTURING CELLS, AND CRYOPRESERVATION SUBSTRATE

(57) The present invention provides a method for cryopreserving cells, including cryopreserving cells for 30 days or more in a state in which the cells are seeded on a porous membrane without using liquid nitrogen. The present invention further provides a cell culture method including a step of cryopreserving cells seeded on a porous membrane for 30 days or more without using liquid nitrogen. Further, the present invention provides a cryopreservation substrate formed of a porous membrane for use in cryopreserving seeded cells for 30 days or more.

EP 4 729 607 A1

## Description

[Technical Field]

The present invention relates to a method for cryopreserving cells, a cell culture method, and a cryopreservation substrate, and particularly relates to a technique for cryopreserving cells in a state in which the cells are seeded on a porous membrane.

[Background Art]

In recent years, studies using cells, such as production of in vivo proteins such as vaccines, enzymes, hormones, antibodies, and cytokines, virus-producing cells, and cells used in regenerative medicine have been actively conducted. In particular, it is known that a cell culture substrate serving as a scaffold for culturing cells has various effects on cells, for example, the differentiation capacity of stem cells changes due to its stiffness (Non Patent Literature 1), and the cell culture substrate attracts a great deal of attention.

As a technique related to a cell culture substrate, for example, there have been proposed techniques such as a technique using a nonwoven fabric subjected to a special surface treatment as a substrate (Patent Literature 1), a technique using a nanofiber as a substrate (Patent Literature 2), a technique using a polyimide porous membrane as a substrate (Patent Literature 3), and a technique using a hollow microfiber as a substrate (Patent Literature 4). These documents all suggest that cryopreservation is possible under cryogenic conditions (about -196°C) using liquid nitrogen in a state in which cells are seeded on a cell culture substrate.

Although the cryogenic conditions using liquid nitrogen disclosed in Patent Literatures 1 to 4 have little influence on cells, use of special equipment and liquid nitrogen which is easily volatilized involves difficulty in management and procurement thereof, as well as complicated temperature management and increased cost.

In view of these problems, techniques for cryopreservation at higher temperatures have been studied. For example, a technique is known in which cryopreservation is performed at a temperature around -80°C using an ultra-low temperature freezer, dry ice, or the like. However, since the temperature around -80°C has a large influence on the cells as compared with the cryogenic conditions using liquid nitrogen, the temperature is limited to short-term preservation or short distance transportation, and thus is not suitable for long-term preservation of cells.

Therefore, as a technique capable of preserving cells for a long period of time even in a relatively high temperature zone around -80°C, a technique related to improvement in a medium composition or a cryoprotective solution has been proposed (Non Patent Literature 2 and Patent Literature 5).

[Citation List]

[Patent Literatures]

[Patent Literature 1] JP H6-209767 A
[Patent Literature 2] WO 2014/196549
[Patent Literature 3] WO 2016/121767
[Patent Literature 4] JP 2020-171317 A
[Patent Literature 5] JP 2020-039326 A

[Non Patent Literatures]

[Non Patent Literature 1] Cell 2006, 126, 677-689 [Non Patent Literature 2] Scientific Reports 2016, 6, 1-13

[Summary of Invention]

[Technical Problem]

The techniques disclosed in Non Patent Literature 2 and Patent Literature 5 are performed in a suspension state even in the case of adhesive cells. In this technique of cryopreserving in a suspension state, cells cultured in a dish, a bottle, or the like are detached by, for example, trypsin treatment, centrifuged and collected, and then repeatedly washed and centrifuged. Thereafter, the cells are further placed in a preservation tube or the like, the tube is filled with a preservation solution, and the cells are frozen in an ultra-low temperature freezer or the like and then preserved in liquid nitrogen. As described above, not only the operation becomes complicated, but also the trypsin treatment and the washing have a problem that stress is applied to the cells.

In addition, the method for cryopreservation in a suspension state also has a problem that washing and centrifugation are required to remove the cell preservation solution and replace the cell preservation solution with the culture solution in thawing and re-culturing, and the cells are stressed again.

On the other hand, the techniques disclosed in Patent Literatures 1 to 4 are merely focused on adhesion between cells and a cell culture substrate, and only suggest that cells can be cryopreserved even in a cryogenic environment using liquid nitrogen. Therefore, the preservation period for cryopreservation is only a short period of several days to 20 days. That is, there has been no attempt to solve the problems related to the preservation temperature and the preservation period at which cells are cryopreserved by focusing on the cell culture substrate.

The present invention has been made in view of such a situation, and an object of the present invention is to provide a method capable of performing cryopreservation for a long period of time inexpensively and easily without impairing the function and the like of cells. Another object is to provide a method for culturing cells which have been cryopreserved for a long period of time. Yet another object is to provide a cryopreservation substrate for long-term cryopreservation.

[Solution to Problem]

As a result of intensive studies, the present inventors have surprisingly found a relationship between a cell culture substrate and cryopreserved cells. That is, the present inventors have found that, by using a porous membrane as a cryopreservation substrate as a cell cryopreservation method in place of conventional cryopreservation with liquid nitrogen under cryogenic conditions, freezing and thawing operations are simplified, and cells can be stably cryopreserved for a long period of time even under higher temperature conditions without using liquid nitrogen, and thus have completed the present invention. In addition, the present inventors have completed an invention related to a method for culturing cells, and an invention related to a cryopreservation substrate formed of a porous membrane, in which even cells thawed after long-term cryopreservation can be stably cultured without impairing the functions thereof and the like, by using a porous membrane as a cryopreservation substrate.

That is, the present invention provides the following.

[1] A method for cryopreserving cells, including cryopreserving cells for 30 days or more in a state in which the cells are seeded on a porous membrane without using liquid nitrogen.

[2] The method according to [1], wherein the cells are animal cells.

[3] The cell cryopreservation method according to [1] or [2], wherein the porous membrane is a polymer porous membrane.

[4] The method according to [3], wherein an average pore size of a B surface of the polymer porous membrane is larger than an average pore size of an A surface of the polymer porous membrane.

[5] The method according to [3] or [4], wherein the B surface has an average pore size of 10 $\mu$m or more.

[6] The method according to any one of [3] to [5], wherein the A surface has an average pore size of 0.01 $\mu$m or more.

[7] The method according to any one of [1] to [6], wherein the porous membrane has an average pore size of 0.01 $\mu$m or more and 500 $\mu$m or less.

[8] The method according to any one of [3] to [7], wherein the polymer porous membrane is a polyimide porous membrane or a polyethersulfone porous membrane.

[9] The method according to any one of [3] to [8], wherein the porous membrane is a porous membrane provided in a cell culture insert.

[10] The method according to any one of [2] to [9], wherein the animal cells are virus-producing cells or hybridomas.

[11] The method according to any one of [2] to [9], wherein the animal cells are mesenchymal stem cells.

[12] The method according to any one of [1] to [11], wherein a temperature for cryopreservation is -150°C or higher.

[13] A cell culture method including the steps of:

(1) step: seeding cells on a porous membrane;
(2) step: cryopreserving the cells seeded on the porous membrane for 30 days or more without using liquid nitrogen;
(3) step: thawing the cells seeded on the porous membrane; and
(4) step: culturing the thawed cells.

[14] The method according to [13], wherein the porous membrane is a porous membrane provided in a cell culture insert.

[15] A cryopreservation substrate formed of a porous membrane for use in cryopreserving seeded cells for 30 days or more.

[16] The cryopreservation substrate according to [15], wherein the porous membrane is a porous membrane provided in a cell culture insert.

[Advantageous Effect of Invention]

According to the present invention, it has become possible to cryopreserve cells for an unparalleled long period of time while maintaining the function of the cells without deterioration without using liquid nitrogen. As a result, long-distance transportation has become possible regardless of whether it is domestic or foreign transportation. In addition, since expensive liquid nitrogen and special equipment are unnecessary, the cost can be greatly reduced as compared with the conventional cryogenic conditions. From another point of view, since freezing, preserving, and thawing operations can be performed while cells are attached to the porous membrane, the operation is simplified. From still another point of view, cryopreservation and cell culture can be performed without exchanging the cell culture substrate and the freezing substrate, thus making it possible to cryopreserve cells simply and inexpensively without applying a load to the cells.

[Brief Description of Drawings]

Fig. 1 is a graph relating to virus titer of cells cryopreserved in an ultra-low temperature freezer.
Fig. 2 is a graph relating to virus titer of cells cryopreserved using liquid nitrogen.
Fig. 3 is a view showing a state in which a cell culture insert is inserted into a well.

[Description of Embodiments]

In the present specification, terms related to the present invention are defined, and each invention will be described in detail thereafter.

First, terms related to the present invention will be described.

The term "porous membrane" in the present invention refers to a membrane (film) having a large number of small voids inside or on the surface, and the material for forming the porous membrane is not particularly limited, and may be an inorganic substance, an organic substance, or a composite thereof. However, the "porous membrane" is a concept not including so-called "woven fabric" or "nonwoven fabric" most of which is formed of fibers. On the other hand, the "porous membrane" is a concept including a porous membrane containing fibers as accessory components (for example, less than 30 wt% with respect to the entire porous membrane). Of course, a laminate of a porous membrane and a nonwoven fabric or the like relates to the present invention because a porous membrane is used.

The phrase "without using liquid nitrogen" in the present invention means that liquid nitrogen is not used as a refrigerant at freezing and/or preserving, and the use of liquid nitrogen in other processes is not included in the concept of "without using liquid nitrogen". In particular, it is preferred not to use liquid nitrogen even at the processes other than freezing and/or preserving.

The term "seeding" in the present invention refers to a state in which cells are in contact with a substrate or an action thereof, and preferably refers to a state in which cells are adhered to or cultured on a substrate (such as a porous membrane) or an action thereof.

Regarding the terms "A surface" and "B surface" in the present invention, one of the two main surfaces of the porous membrane is referred to as A surface, and the other surface is referred to as B surface. That is, the "A surface" and "B surface" refer to the main surfaces for convenience, and do not represent the front surface or the back surface.

Next, the cryopreservation method, the cell culture method, and the cryopreservation substrate of the present invention will be described in order.

I. Cryopreservation method

In the cryopreservation method of the present invention, cells are cryopreserved for 30 days or more in a state in which the cells are seeded on a porous membrane without using liquid nitrogen. In the present specification, for convenience of description, cells, a porous membrane, and a cryopreservation method are described in this order.

1. Cells

The cell applicable to the present invention is not particularly limited. This is because the method of the present invention imposes little stress on cells. Examples of the cells include animal cells, insect cells, plant cells, yeast cells, and bacteria.

Animal cells are largely divided into cells from animals belonging to the subphylum Vertebrata, and cells from non-vertebrates (animals other than animals belonging to the subphylum Vertebrata), but the source of the animal cells is not particularly limited herein.

For example, the subphylum Vertebrata includes the superclass Agnatha and the superclass Gnathostomata, and examples of the superclass Gnathostomata include the class Mammalia, the class Aves, the class Amphibia, and the class Reptilia. They are preferably cells derived from animals belonging to the class Mammalia, generally known as mammals. Examples of the mammals include, preferably, mice, rats, humans, monkeys, pigs, dogs, sheep and goats.

There are no particular restrictions on the source of the plant cells, for the purpose of the present specification. For example, cells of plants including bryophytes, pteridophytes and spermatophytes are targeted.

Plants from which spermatophyte cells are derived include both monocotyledons and dicotyledons. Examples of the monocotyledons include, but are not limited to, Orchidaceae plants, Poaceae plants (rice, corn, barley, wheat, sorghum, and the like), and Cyperaceae plants. Examples of the dicotyledons include plants belonging to many subclasses including the subclass Asteridae, the subclass Magnoliidae, and the subclass Rosidae.

Algae can be regarded as cell-derived organisms. For example, algae include different groups from cyanobacteria (blue-green algae) that are eubacteria to those that are eukaryotic unicellular organisms (diatoms, yellow-green algae, dinoflagellates, and the like), and marine algae that are multicellular organisms (red algae, brown algae, and green algae).

The types of archaebacteria and bacteria herein are also not particularly limited. The archaebacteria are composed of a group consisting of methanogenic bacteria, extreme halophilic bacteria, thermophilic acidophilic bacteria, hyperthermophilic bacteria, and the like. The bacteria can be selected from the group consisting of, for example, lactic acid bacteria, *Escherichia coli*, *Bacillus subtilis*, cyanobacteria, and the like.

As described above, in the method of the present invention, the types of animal cells or plant cells that may be used are not particularly restricted, but are preferably selected from the group consisting of hybridomas, pluripotent stem cells, tissue stem cells, somatic cells, and germ cells.

In the present specification, the term "hybridoma" refers to a cell that produces an antibody and is obtained by cell fusion of B cells obtained by immunizing an animal other than human with an antigen and myeloma cells. The source of the B cells and myeloma cells is not particularly limited, and examples thereof include mice (including nude mice), rats, guinea pigs, rabbits, goats, sheep, and chickens.

Throughout the present specification, the term "pluripotent stem cells" refers to a general term for stem cells having the ability to differentiate into cells of a variety of tissues (pluripotent differentiating capabilities). While not a restriction, examples of the pluripotent stem cells include embryonic stem cells (ES cells), induced pluripotent stem cells (iPS cells), embryonic germ cells (EG cells) and germ stem cells (GS cells). Preferably, ES cells or iPS cells can be exemplified. Particularly preferred are iPS cells, which are free of ethical problems, for example. Any publicly known pluripotent stem cells may be used, for example, the pluripotent stem cells described in WO 2009/123349 (PCT/JP2009/057041) may be used.

In the present specification, the term "tissue stem cells" refers to stem cells that are cell lineage capable of differentiation but only to limited specific tissues, though having the ability to differentiate into a variety of cell types (pluripotent differentiating capabilities). For example, hematopoietic stem cells in the bone marrow are the source of blood cells, while neural stem cells differentiate into neurons. Additional types include hepatic stem cells from which the liver is formed and skin stem cells that form skin tissue. Preferably, the tissue stem cells can be selected from among mesenchymal stem cells, hepatic stem cells, pancreatic stem cells, neural stem cells, skin stem cells or hematopoietic stem cells, and the like.

In the present specification, the term "somatic cells" refers to cells other than germ cells, among the cells composing a multicellular organism. Preferably, the somatic cells can be selected from among hepatocytes, pancreatic cells, muscle cells, bone cells, osteoblasts, osteoclasts, chondrocytes, adipocytes, skin cells, fibroblasts, pancreatic cells, renal cells, lung cells, or blood cells such as lymphocytes, erythrocytes, leukocytes, monocytes, macrophages or megakaryocytes.

In the present specification, the term "germ cells" refers to cells having the role of passing on genetic information to the succeeding generation in reproduction. Examples thereof include gametes for sexual reproduction, i.e., the ova, egg cells, sperm, sperm cells, and spores for asexual reproduction.

In addition, the cells may also be selected from the group consisting of sarcoma cells, established cell lines and transformants.

In the present specification, the term "sarcoma" refers to cancer occurring in non-epithelial cell-derived connective tissue cells, such as the bone, cartilage, fat, muscle or blood, and includes soft tissue sarcomas, malignant bone tumors and the like. Sarcoma cells are cells derived from sarcoma.

In the present specification, the term "established cell line" refers to cultured cells that are maintained in vitro for a long period of time and reach a stabilized character and can be semi-permanently subcultured. Existing cell lines include those derived from various tissues of various species including humans, such as PC12 cells (from rat adrenal medulla), CHO cells (from Chinese hamster ovary), HEK293 cells (from human embryonic kidney), HL-60 cells from (human leukocytes) and HeLa cells (from human cervical cancer), Vero cells (from African green monkey kidney epithelial cells), MDCK cells (from canine kidney epithelial cells), HepG2 cells (from human hepatic cancer), and pig kidney-derived established cell lines (CPK), and these can be used. Even virus-producing cells, for example, those obtained by infecting established cell lines with various viruses (virus-producing established cell lines) or established cell lines into which vectors that produce various viruses (virus-producing vectors) are introduced can be cryopreserved for a long period of time.

In the present specification, the term "transformants" refers to cells with an altered genetic nature created by extracellularly introduced nucleic acid (DNA and the like). For transformation of animal cells, plant cells, and bacteria, there are known suitable methods, and the transformed cells can also be used.

In the method of the present invention, any cell can be cryopreserved for a long period of time, and among them, preferably, hybridomas, pluripotent stem cells, tissue stem cells, or established cell lines can be selected, and in particular, hybridomas, mesenchymal stem cells, established cell lines, or virus-producing cells can be selected. In an embodiment, in the method of the present invention, it is possible to cryopreserve a single layer of cells or tissues or a plurality of layers (for example, 2 to 5 layers, 2 to 4 layers, or 2 to 3 layers) of cells or tissues for a long period of time. In the present specification, the term "tissue" is a structure formed by accumulation of a plurality of cells, and examples thereof include skin tissue (for example, hair associated skin tissue), myocardial tissue, skeletal muscle tissue, smooth muscle tissue, liver tissue, renal tissue, digestive tract tissue, eye tissue (for example, corneal tissue), brain tissue, thymus tissue, testicular tissue, pancreatic tissue, thyroid tissue, mammary tissue, salivary gland tissue, and lung tissue. The "tissue" may be a sheet-like tissue (for example, a cell sheet) formed by culturing cells derived from the tissue, or may be a sheet-like tissue isolated from a biological tissue.

## 2. Porous membrane

The porous membrane according to the present invention mainly functions as a substrate for seeding cells in cryopreservation. The porous membrane refers to a membrane (film) having a large number of small voids inside or on the surface, and the material for forming the porous membrane is not particularly limited, and may be an inorganic substance such as ceramics, an organic substance such as a polymer porous membrane, or a composite thereof. Hereinafter, as an embodiment, a polymer porous membrane formed of a polymer will be described in detail as an example.

## 2-1. Polymer porous membrane

In the present specification, the "polymer porous membrane" has a surface layer A (hereinafter, may be referred to as "A surface" or "mesh surface") and a surface layer B (hereinafter, may be referred to as "B surface" or "large pore surface"). The shapes of the pores of the A surface and the B surface may be the same or different. The pore sizes (average pore sizes) of the A surface and the B surface are not particularly limited, but the average pore size of the pores present in the B surface is preferably larger than the average pore size of the pores present in the A surface.

The average pore size (hereinafter, may be referred to as "average fine pore size") of the pores present in the A surface is not particularly limited, but is, for example, preferably 0.01 $\mu$m or more, more preferably 0.05 $\mu$m or more, still more preferably 0.1 $\mu$m or more, particularly preferably 0.5 $\mu$m or more. The upper limit thereof is preferably less than 200 $\mu$m, more preferably 150 $\mu$m or less, still more preferably 100 $\mu$m or less, and particularly preferably 50 $\mu$m or less, 40 $\mu$m or less, 30 $\mu$m or less, 20 $\mu$m, or 15 $\mu$m or less.

The average pore size of the pores present in the B surface is preferably larger than the average pore size of the pores present in the surface layer A. For example, the average pore size of the pores present in the B surface is preferably more than 5 $\mu$m, more preferably 20 $\mu$m or more, still more preferably 30 $\mu$m or more, particularly preferably 50 $\mu$m or more, or 60 $\mu$m or more. The upper limit thereof is preferably 200 $\mu$m or less, and more preferably 100 $\mu$m or less.

The average pore size of the pores present in the surface layer of the polymer porous membrane can be determined by measuring the pore areas of 200 or more opening portions from a scanning electron micrograph of the porous membrane surface, and calculating the average diameter when the shape of the pore is a perfect circle, according to the following Equation (1) from the average value of the pore areas.

[Math. 1]

$$\text{Average pore size} = 2 \times \sqrt{(S_a / \pi)} \qquad (1)$$

(wherein, Sa represents the average value of pore areas.)

The thicknesses of the surface layer A and the surface layer B may be the same or different, and are not particularly limited. For example, the thicknesses are each preferably 0.01 or more, and the upper limits thereof are preferably 50 $\mu$m or less, and more preferably 20 $\mu$m or less.

The polymer porous membrane used in the present invention may have an intermediate layer between the A surface and the B surface. The intermediate layer may be a solid layer having no pores or a porous layer having pores. When the intermediate layer has pores, the intermediate layer may be the same as or different from the surface layer of the polymer porous membrane. Preferably, the intermediate layer is a porous layer, and the porous layer forms a macrovoid layer.

A macrovoid layer in the case where the intermediate layer is a porous layer will be described as an example. In the present specification, the term "macrovoid layer" refers to a membrane having an average pore size of 10 $\mu$m or more in the planar direction of the membrane of the layer. The average pore size of the macrovoids in the macrovoid layer in the planar direction of the membrane is not particularly limited, but for example, the upper limit of the average pore size is preferably 500 $\mu$m or less, more preferably 100 $\mu$m or less, and particularly preferably 80 $\mu$m or less. The thickness of the partition wall in the macrovoid layer (the frame forming the pore in the macrovoid layer) is not particularly limited, but for example, is preferably 0.01 $\mu$m or more, and the upper limit thereof is preferably 50 $\mu$m or less, and more preferably 20 $\mu$m or less.

In an embodiment, at least one partition wall in the macrovoid layer may have pores connecting neighboring macrovoids. The average pore size of the communicating pores is preferably 0.01 or more, and the upper limit thereof is preferably 100 $\mu$m or less, and more preferably 50 $\mu$m or less. The partition wall may have one or more communicating pores. In another embodiment, the partition wall in the macrovoid layer is not required to have pores.

The total film thickness (total film thickness of the A surface and the B surface, and if present, the intermediate layer) of the polymer porous membrane is not particularly limited, but may be 5 $\mu$m or more, 10 $\mu$m or more, 20 $\mu$m or more, or 25 $\mu$m or more, and may be 500 $\mu$m or less, 300 $\mu$m or less, 100 $\mu$m or less, 75 $\mu$m or less, or 50 $\mu$m or less. The total film thickness is preferably 5 to 500 $\mu$m, and more preferably 10 to 100 $\mu$m.

In the present specification, the method for measuring the film thickness of the polymer porous membrane can be performed using a contact thickness gauge.

In the present specification, the porosity of the polymer porous membrane is not particularly limited, but is, for example, preferably 40% or more, more preferably 50% or more, and still more preferably 60% or more, and the upper limit thereof is preferably less than 95%, more preferably 90% or less, and still more preferably 85% or less.

In the present specification, the porosity of the polymer porous membrane can be determined by measuring the film thickness and mass of the polymer porous membrane cut out to a prescribed size, and performing calculation from the basis weight according to the following Equation (2).

[Math. 2]

$$\text{Porosity (\%)} = (1 - w / (S \times d \times D)) \times 100 \qquad (2)$$

(wherein S represents the area of the polymer porous membrane, d represents the total film thickness, w represents the measured mass, and D represents the polymer density. The density is defined as 1.34 g/cm$^3$ when the polymer is a polyimide.)

In the present specification, the polymer porous membrane is preferably a polymer porous membrane having a three-layer structure including a surface layer A and a surface layer B having a plurality of pores, and a macrovoid layer sandwiched between the surface layer A and the surface layer B. Here, the average pore size of the pores present in the surface layer A is preferably 0.01 $\mu$m or more and 15 $\mu$m or less, and the average pore size of the pores present in the surface layer B is preferably 20 $\mu$m or more and 100 $\mu$m or less. The macrovoid layer has a partition wall joined to the surface layers A and B and a plurality of macrovoids surrounded by the partition wall and the surface layers A and B, the thickness of the partition wall of the macrovoid layer and the surface layers A and B is preferably 0.01 $\mu$m or more and 20 $\mu$m or less, the pores in the surface layers A and B communicate with the macrovoids, and the total film thickness is preferably 5 $\mu$m or more and 500 $\mu$m or less. The porosity of the polymer porous membrane is preferably 40% or more and less than 95%. In an embodiment, at least one partition wall in the macrovoid layer has one or more pores connecting neighboring macrovoids with each other and having an average pore size of 0.01 or more and 100 $\mu$m or less, preferably 0.01 or more and 50 $\mu$m or less. In another embodiment, the partition wall does not have such pores.

In the present specification, the shape of the polymer porous membrane is not particularly limited, and for example, the size of the polymer porous membrane can be adjusted according to the shape of a culture container or a cryopreservation container to be used.

In the present specification, the polymer porous membrane can be used in a state of being placed in a culture dish or the like as a membrane alone, but for example, the polymer porous membrane may also be used in a state in which a part of the membrane is fixed to a resin or a metal, or in a state in which the polymer porous membrane is housed in an appropriate casing as described in WO 2018/021368.

In the present specification, the polymer porous membrane is preferably sterilized. The sterilization treatment is not particularly limited, and any sterilization treatment can be selected from dry heat sterilization, ethylene oxide gas sterilization, high pressure steam sterilization, sterilization with a disinfectant such as ethanol, and sterilization with electromagnetic waves such as ultraviolet rays and gamma rays, and/or radiation. It is also possible to select and appropriately combine a plurality of these sterilization methods.

In the present specification, the polymer porous membrane preferably has the structural features described above, but a polyimide porous membrane formed of a polyimide porous material or a polyethersulfone porous membrane formed of polyethersulfone (PES) can be particularly preferably used.

By using the porous membrane as described above as a substrate for cryopreservation, the effect of the cryopreservation method of the present invention can be exhibited.

3. Cell culture insert

In an embodiment of the present invention, a cell culture insert including a cylindrical container and the above-described porous membrane disposed at the bottom of the cylindrical container can be applied as a substrate for cryopreservation. The cell culture insert is hereinafter also referred to as "cell culture insert of the present invention". In the present specification, the description "cell culture insert" can be simply described as "insert", and both descriptions can be used interchangeably to indicate the same meaning.

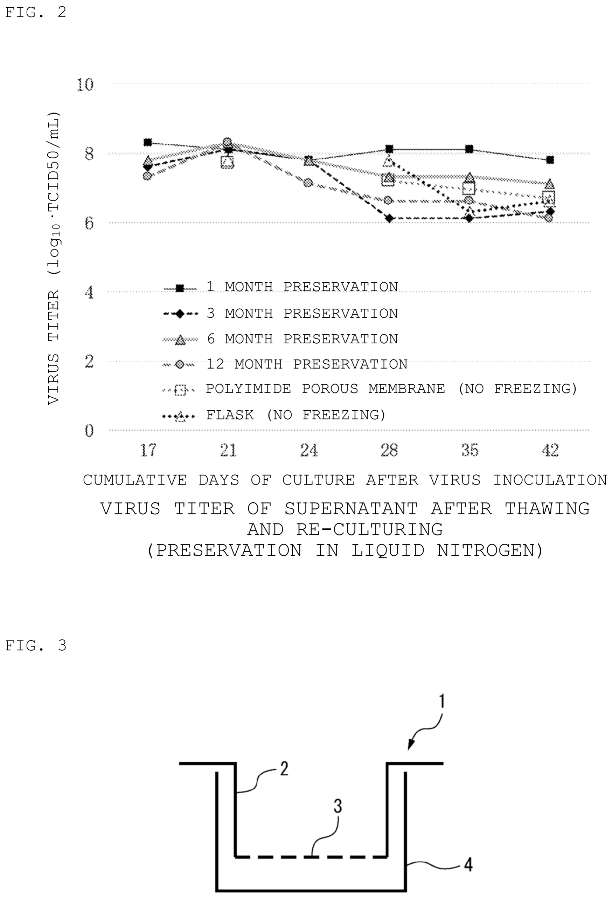

As used herein, the term "cell culture insert" refers to an insert for cell culture that is inserted inside each well of a dish or multi-well plate and used commonly in cell culture. A common insert is a cylindrical container having a size that can be inserted into a dish or well, and has a bottom and a side surface to ensure a constant volume that enables cell culture. According to the present invention, a cell culture insert is provided by disposing the above-described porous membrane at the bottom of the cylindrical container. Here, the cylindrical container has a space in which cell culture can be performed inside the cell culture insert, and has a size to be inserted into a general dish or well. The upper surface of the cylindrical container preferably has an arm structure that can be hooked on the upper surface of a dish or well so that the cylindrical container does not come into contact with the bottom of the dish or well. The cylindrical container is not limited as long as it has the above-described shape, and containers that are generally used, widely used, or commercially available can be used. A cell culture insert is provided by bonding a porous membrane to the bottom surface of the cylindrical container. Alternatively, a cell culture insert obtained by using a commonly used, widely used or commercially available insert including a membrane disposed therein, and substituting the membrane with the porous membrane applied in the present invention may also be used. The porous membrane used for the present invention can be easily formed according to the size and shape of the bottom of the insert to be used. Although not particularly limited, a schematic view when the cell culture insert is inserted into the well is shown in Fig. 3.

The bottom of the insert generally has a shape such as a circle or an ellipse, and if the bottom has a circular shape, the diameter of the circular bottom is less than or equal to the diameter of the opening of the upper part of the dish or well. Therefore, the diameter of the porous membrane to be disposed at the bottom is appropriately adjusted to be less than or equal to the opening diameter of the dish (for example, a nominal diameter of 35 mm, a nominal diameter of 60 mm, a nominal diameter of 100 mm, a nominal diameter of 150 mm, and the like) or well (for example, 6 wells, 12 wells, 24 wells, 96 wells, and the like) into which the cell culture insert is to be inserted and used. The nominal diameter of the dish is common, and when the nominal diameter is 100 mm, the actual diameter of the dish is around 80 mm, and the nominal diameter does not indicate an accurate diameter accordingly. For example, the diameter of the porous membrane is typically 3 to 130 mm, which is the size to fit in a dish or well.

The insert used for the present invention is preferably sterilized. The sterilization treatment is not particularly limited, and any sterilization treatment can be selected from dry heat sterilization, ethylene oxide gas sterilization, high pressure steam sterilization, sterilization with a disinfectant such as ethanol, and sterilization with electromagnetic waves such as ultraviolet rays and gamma rays, and/or radiation. It is also possible to select and appropriately combine a plurality of these sterilization methods.

4. Cryopreservation method

The cryopreservation method according to the present invention can be performed by a method similar to the conventional method except that a porous membrane (or a cell culture insert provided with a porous membrane) to which cells are attached and carried is used, and detachment of cells or an operation using a centrifuge is unnecessary. For example, a porous membrane (or a cell culture insert provided with a porous membrane) to which cells are attached and carried is transferred from a culture solution into a cryopreservation container containing a cryoprotective solution, the cells are slowly frozen in an ultra-low temperature freezer (for example, a deep freezer at about -80°C or the like), and then the cells can be cryopreserved at a target temperature. Alternatively, the culture solution is removed from the container, the cryoprotective solution is introduced in the container, the cells are slowly frozen in an ultra-low temperature freezer, and then the cells can be cryopreserved at a target temperature. The cells may also be appropriately washed with, for example, PBS or the like during these operations. In an embodiment, when a cell culture insert carrying the cells or tissues is cryopreserved, the cryoprotective solution is introduced only inside the cell culture insert or inside and outside the cell culture insert, and the cells or tissues are frozen in an ultra-low temperature freezer (for example, a deep freezer at about -80°C or the like), and then may be further cryopreserved at a target temperature.

In the present specification, the phrase "higher temperature conditions without using liquid nitrogen" is a temperature condition higher than that for liquid phase preservation or gas phase preservation using liquid nitrogen, and the lower limit thereof is -150°C or higher, preferably -120°C or higher, and more preferably -90°C or higher.

In order to achieve such a temperature, for example, the set temperature of the ultra-low temperature freezer may be set to -150°C or higher, -120°C or higher, -100°C or higher, or -80°C.

In the "higher temperature conditions without using liquid nitrogen", the upper limit thereof may be, for example, -10°C or lower, -20°C or lower, -30°C or lower, - 40°C or lower, -50°C or lower, -60°C or lower, or -70°C or lower.

In an embodiment of the present invention, the "higher temperature conditions without using liquid nitrogen" may be a range in which the lower limit and the upper limit are arbitrarily combined, and the set temperature may be, for example, -150°C to -10°C, -150°C to -20°C, -150°C to -30°C, -150°C to -40°C, -150°C to -50°C, - 150°C to -60°C, or -150°C to -70°C. For example, the temperature may be -120°C to -10°C, -120°C to -20°C, -120°C to -30°C, -120°C to -40°C, -120°C to -50°C, -120°C to - 60°C, or -120°C to -70°C. For example, the temperature may be -100°C to -10°C, -100°C to -20°C, -100°C to -30°C, - 100°C to -40°C, -100°C to -50°C, -100°C to -60°C, or -100°C to -70°C. For example, the temperature may be -90°C to - 10°C, -90°C to -20°C, -90°C to -30°C, -90°C to -40°C, -90°C to -50°C, -90°C to -60°C, or -90°C to -70°C.

In the present specification, the phrase "long period of time" in the cryopreservation period of cells refers to a period of more than 1 month (or 30 days), preferably a period of more than 3 months (or 90 days), more preferably a period of more than 6 months (or 180 days), and more preferably a period of more than 12 months (or 365 days). Also, in the present specification, the "long period of time" in the cryopreservation period of cells may be a period of more than at least 1 month (or at least 30 days), preferably at least 3 months (or at least 90 days), more preferably at least 6 months (or at least 180 days), and more preferably at least 12 months (or at least 365 days).

As described above, the cryopreservation method of the present invention can be very simply carried out because the method does not use liquid nitrogen. The cryopreservation method of the present invention has not only an advantage that the load on the cells to be cryopreserved can be reduced, but also an advantage that the method can be applied to any aspect of the cryopreservation method. A special protective solution as in Non Patent Literature 2 or Patent Literature 5 may be used, but the cryopreservation method of the present invention is excellent particularly in view of enabling cryopreservation without causing deterioration while maintaining the function of cells by using, for example, a commercially available cryoprotective solution (cell freezing solution) without using a protective solution having a special composition.

II. Cell culture method

The cell culture method of the present invention is a method including the following steps:

(1) step: seeding cells on a porous membrane;
(2) step: cryopreserving the cells seeded on the porous membrane for 30 days or more without using liquid nitrogen;
(3) step: thawing the cells seeded on the porous membrane; and
(4) step: culturing the thawed cells.

The (1) step is a step of seeding cells before being cryopreserved on a porous membrane. Preferably, cells to be cryopreserved can be cultured by using a porous membrane as a substrate. Here, the cells may be simply adhered to the porous membrane, or the cells may be cultured. It can be appropriately selected according to the type and amount of cells to be cryopreserved.

As the cell culture method, a known method can be adopted, and the culture conditions can be appropriately determined according to the type of cells and the like. Culture methods suitable for each of animal cells, plant cells, and bacteria are known, and a person skilled in the art can culture the cells on the polymer porous membrane by any known method. The cell culture medium can also be appropriately prepared according to the type of cells.

The cell culture method and the cell culture medium for animal cells are described, for example, in a cell culture medium catalog of Lonza Group AG. The cell culture method and the cell culture medium for plant cells are described, for example, in plant tissue medium series of FUJIFILM Wako Pure Chemical Corporation. The cell culture method and the cell culture medium for bacteria are described, for example, in a general bacterial medium catalog of Becton, Dickinson and Company. The cell culture medium used for the method of the present invention may be in any form of a liquid medium, a semi-solid medium, a solid medium, or the like. In addition, by spraying a liquid medium in the form of droplets into the cell culture container, the medium may be brought into contact with the polymer porous membrane carrying the cells.

The (2) step is a step of cryopreserving the cells seeded on the porous membrane, and the details are as described in the cryopreservation method. In order to freeze the cells, so-called slow cooling may be adopted. Slow cooling is preferred because the water present outside the cells freezes before formation of ice in the cells. In this step, the technique of Non Patent Literature 2 or Patent Literature 5 may be used, but the present invention is excellent in view of enabling cryopreservation without causing deterioration while maintaining the function of the cells without using these techniques.

The (3) step is a step of thawing the cryopreserved cells. This step can be performed by a method similar to the conventional method except that a washing operation using a centrifuge or the like is unnecessary.

The (4) step is a step of culturing the thawed cells (hereinafter, also referred to as "re-culture"). This step can be performed by a method similar to the conventional method except that a washing operation using a centrifuge and cell seeding are unnecessary. Although not particularly limited, for example, after the cryoprotective solution is thawed, the polymer porous membrane to which the cells are attached and carried is transferred from the cryoprotective solution to the culture solution, and the culture can be resumed. Conversely, the cryoprotective solution may be removed after thawing, and the culture solution may be added to restart the culture. The cells may also be appropriately washed with, for example, PBS or the like during these operations.

III. Cryopreservation substrate

The cryopreservation substrate of the present invention is meaningful in that a porous membrane is selectively used as a substrate. Details of the porous membrane, particularly details of the polymer porous membrane are as described above. Here, a production method will be described in detail by using a polyimide porous membrane and a polyethersulfone porous membrane as an example of the polymer porous membrane.

III-1. Polyimide porous membrane

In the present specification, the term "polyimide" refers to a general term for polymers containing imide bonds in the repeating unit, preferably polymers containing 50 mol% or more of imide bonds in all repeating units, and usually refers to an aromatic polyimide in which aromatic compounds are directly linked by imide bonds. An aromatic polyimide has an aromatic-aromatic conjugated structure via an imide bond, and therefore has a strong rigid molecular structure, and since the imide bonds provide powerful intermolecular force, it has very high levels of thermal, mechanical and chemical properties.

The polyimide porous membrane which may be used for the present invention is a polyimide porous membrane that preferably contains polyimide as a main component and is obtained from tetracarboxylic dianhydride and diamine, more preferably a polyimide porous membrane composed of tetracarboxylic dianhydride and diamine. The phrase "including as the main component" means that it essentially contains no components other than the polyimide obtained from a tetracarboxylic dianhydride and a diamine, as constituent components of the polyimide porous membrane, or that it may contain them but they are additional components that do not affect the properties of the polyimide obtained from the tetracarboxylic dianhydride and diamine.

A polyamic acid is obtained by polymerization of a tetracarboxylic acid component and a diamine component. A polyamic acid is a precursor for forming polyimide that can be cyclized to a polyimide by thermal imidization or chemical imidization.

The polyamic acid used may be any one that does not have an effect on the present invention, even if a portion of the amic acid is imidized. Specifically, the polyamic acid may be partially thermally imidized or chemically imidized.

When the polyamic acid is to be thermally imidized, there may be added to a solution (hereinafter, also referred to as "polyamic acid solution") in which the polyamic acid is dissolved, if necessary, additives such as an imidization catalyst, an organic phosphorus-containing compound, or fine particles such as inorganic fine particles or organic fine particles. In addition, when the polyamic acid is to be chemically imidized, there may be added to the polyamic acid solution, if necessary, additives such as a chemical imidization agent, a dehydrating agent, or fine particles such as inorganic fine particles or organic fine particles.

In an embodiment, the polyimide porous membrane which may be used for the present invention includes a colored polyimide porous membrane obtained by forming a polyamic acid solution composition including a polyamic acid solution obtained from a tetracarboxylic acid component and a diamine component, and a coloring precursor, and then heat treating it at 250°C or higher.

In the present specification, a "coloring precursor" is a precursor that generates a colored substance by partial or total carbonization under heat treatment at 250°C or higher.

Coloring precursors which may be used for the production of the polyimide porous membrane are preferably uniformly dissolved or dispersed in a polyamic acid solution or polyimide solution and subjected to thermal decomposition by heat treatment at 250°C or higher, preferably 260°C or higher, even more preferably 280°C or higher and more preferably 300°C or higher, and preferably heat treatment in the presence of oxygen such as air, at 250°C, preferably 260°C or higher, even more preferably 280°C or higher and more preferably 300°C or higher, for carbonization to produce a colored substance, more preferably producing a black colored substance, with carbon-based coloring precursors being most preferred.

The coloring precursor, when being heated, first appears as a carbonized compound, but compositionally it contains other elements in addition to carbon, and also includes layered structures, aromatic crosslinked structures and tetrahedron carbon-containing disordered structures.

Carbon-based coloring precursors are not particularly restricted, and for example, they include tar or pitch such as petroleum tar, petroleum pitch, coal tar and coal pitch, coke, polymers obtained from acrylonitrile-containing monomers, ferrocene compounds (ferrocene and ferrocene derivatives), and the like. Of these, polymers obtained from acrylonitrile-containing monomers and/or ferrocene compounds are preferred, with polyacrylonitrile being preferred as a polymer obtained from an acrylonitrile-containing monomer.

When the coloring precursor is used, the additives described above may be used. In this case, even if the additives are added to the polyamic acid solution, they are preferably added under conditions that do not cause precipitation of the coloring precursor.

Moreover, in another embodiment, examples of the polyimide porous membrane which may be used for the present invention also include polyimide porous membrane which can be obtained by molding a polyamic acid solution derived from a tetracarboxylic acid component and a diamine component followed by heat treatment without using the coloring precursor.

Regardless of whether or not the coloring precursor is used, the polyimide porous membrane may be produced, for example, by casting a polyamic acid solution into a film, the polyamic acid solution being composed of 3 to 60% by mass of polyamic acid having an intrinsic viscosity number of 1.0 to 3.0 and 40 to 97% by mass of an organic polar solvent, immersing the solution in a coagulating solvent containing water as an essential component or bringing the solution into contact with the coagulating solvent to prepare a porous membrane of the polyamic acid, and imidating the porous membrane of the polyamic acid by heat treatment. In this method, the coagulating solvent containing water as an essential component may be water, or a mixed solution containing 5% by mass or more and less than 100% by mass of water and more than 0% by mass and 95% by mass or less of an organic polar solvent. Further, after the imidation, at least one surface of the resulting polyimide porous membrane may be subjected to plasma treatment.

The tetracarboxylic dianhydride which may be used for the production of the polyimide porous membrane may be any tetracarboxylic dianhydride, selected as appropriate according to the properties desired. Specific examples of tetracarboxylic dianhydrides include pyromellitic dianhydride, biphenyltetracarboxylic dianhydrides such as 3,3',4,4'-biphenyltetracarboxylic dianhydride (s-BPDA) and 2,3,3',4'-biphenyltetracarboxylic dianhydride (a-BPDA), oxydiphthalic dianhydride, diphenylsulfone-3,4,3',4'-tetracarboxylic dianhydride, bis(3,4-dicarboxyphenyl)sulfide dianhydride, 2,2-bis(3,4-dicarboxyphenyl)-1,1,1,3,3,3-hexafluoropropane dianhydride, 2,3,3',4'-benzophenonetetracarboxylic dianhydride, 3,3',4,4'-benzophenonetetracarboxylic dianhydride, bis(3,4-dicarboxyphenyl)methane dianhydride, 2,2-bis(3,4-dicarboxyphenyl)propane dianhydride, p-phenylenebis(trimellitic acid monoester acid anhydride), p-biphenylenebis(trimellitic acid monoester acid anhydride), m-terphenyl-3,4,3',4'-tetracarboxylic dianhydride, p-terphenyl-3,4,3',4'-tetracarboxylic dianhydride, 1,3-bis(3,4-dicarboxyphenoxy)benzene dianhydride, 1,4-bis(3,4-dicarboxyphenoxy)benzene dianhydride, 1,4-bis(3,4-dicarboxyphenoxy)biphenyl dianhydride, 2,2-bis[(3,4-dicarboxyphenoxy)phenyl]propane dianhydride, 2,3,6,7-naphthalenetetracarboxylic dianhydride, 1,4,5,8-naphthalenetetracarboxylic dianhydride, 4,4'-(2,2-hexafluoroisopropylidene)diphthalic dianhydride, and the like. Also preferably used is an aromatic tetracarboxylic acid such as 2,3,3',4'-diphenylsulfonetetracarboxylic acid. These may be used alone or in appropriate combinations of two or more.

Particularly preferred among these are at least one type of aromatic tetracarboxylic dianhydride selected from the group consisting of biphenyltetracarboxylic dianhydride and pyromellitic dianhydride. As a biphenyltetracarboxylic dianhydride there may be suitably used those containing 3,3',4,4'-biphenyltetracarboxylic dianhydride.

As diamine which may be used for the production of the polyimide porous membrane, any diamine may be used. Specific examples of diamines include the following.

1) Benzenediamines with one benzene nucleus, such as 1,4-diaminobenzene(paraphenylenediamine), 1,3-diaminobenzene, 2,4-diaminotoluene and 2,6-diaminotoluene;

2) diamines with two benzene nuclei, including diaminodiphenyl ethers such as 4,4'-diaminodiphenyl ether and 3,4'-diaminodiphenyl ether, and 4,4'-diaminodiphenylmethane, 3,3'-dimethyl-4,4'-diaminobiphenyl, 2,2'-dimethyl-4,4'-diaminobiphenyl, 2,2'-bis(trifluoromethyl)-4,4'-diaminobiphenyl, 3,3'-dimethyl-4,4'-diaminodiphenylmethane, 3,3'-dicarboxy-4,4'-diaminodiphenylmethane, 3,3',5,5'-tetramethyl-4,4'-diaminodiphenylmethane, bis(4-aminophenyl)sulfide, 4,4'-diaminobenzanilide, 3,3'-dichlorobenzidine, 3,3'-dimethylbenzidine, 2,2'-dimethylbenzidine, 3,3'-dimethoxybenzidine, 2,2'-dimethoxybenzidine, 3,3'-diaminodiphenyl ether, 3,4'-diaminodiphenyl ether, 4,4'-diaminodiphenyl ether, 3,3'-diaminodiphenyl sulfide, 3,4'-diaminodiphenyl sulfide, 4,4'-diaminodiphenyl sulfide, 3,3'-diaminodiphenylsulfone, 3,4'-diaminodiphenylsulfone, 4,4'-diaminodiphenylsulfone, 3,3'-diaminobenzophenone, 3,3'-diamino-4,4'-dichlorobenzophenone, 3,3'-diamino-4,4'-dimethoxybenzophenone, 3,3'-diaminodiphenylmethane, 3,4'-diaminodiphenylmethane, 4,4'-diaminodiphenylmethane, 2,2-bis(3-aminophenyl)propane, 2,2-bis(4-aminophenyl)propane, 2,2-bis(3-aminophenyl)-1,1,1,3,3,3-hexafluoropropane, 2,2-bis(4-aminophenyl)-1,1,1,3,3,3-hexafluoropropane, 3,3'-diaminodiphenyl sulfoxide, 3,4'-diaminodiphenyl sulfoxide and 4,4'-diaminodiphenyl sulfoxide;
3) diamines with three benzene nuclei, including 1,3-bis(3-aminophenyl)benzene, 1,3-bis(4-aminophenyl)benzene, 1,4-bis(3-aminophenyl)benzene, 1,4-bis(4-aminophenyl)benzene, 1,3-bis(4-aminophenoxy)benzene, 1,4-bis(3-aminophenoxy)benzene, 1,4-bis(4-aminophenoxy)benzene, 1,3-bis(3-aminophenoxy)-4-trifluoromethylbenzene, 3,3'-diamino-4-(4-phenyl)phenoxybenzophenone, 3,3'-diamino-4,4'-di(4-phenylphenoxy)benzophenone, 1,3-bis(3-aminophenyl sulfide)benzene, 1,3-bis(4-aminophenyl sulfide)benzene, 1,4-bis(4-aminophenyl sulfide)benzene, 1,3-bis(3-aminophenylsulfone)benzene, 1,3-bis(4-aminophenylsulfone)benzene, 1,4-bis(4-aminophenylsulfone)benzene, 1,3-bis[2-(4-aminophenyl)isopropyl]benzene, 1,4-bis[2-(3-aminophenyl)isopropyl]benzene and 1,4-bis[2-(4-aminophenyl)isopropyl]benzene;
4) diamines with four benzene nuclei, including 3,3'-bis(3-aminophenoxy)biphenyl, 3,3'-bis(4-aminophenoxy)biphenyl, 4,4'-bis(3-aminophenoxy)biphenyl, 4,4'-bis(4-aminophenoxy)biphenyl, bis[3-(3-aminophenoxy)phenyl]ether, bis[3-(4-aminophenoxy)phenyl]ether, bis[4-(3-aminophenoxy)phenyl]ether, bis[4-(4-aminophenoxy)phenyl]ether, bis[3-(3-aminophenoxy)phenyl]ketone, bis[3-(4-aminophenoxy)phenyl]ketone, bis[4-(3-aminophenoxy)phenyl]ketone, bis[4-(4-aminophenoxy)phenyl]ketone, bis[3-(3-aminophenoxy)phenyl]sulfide, bis[3-(4-aminophenoxy)phenyl]sulfide, bis[4-(3-aminophenoxy)phenyl]sulfide, bis[4-(4-aminophenoxy)phenyl]sulfide, bis[3-(3-aminophenoxy)phenyl]sulfone, bis[3-(4-aminophenoxy)phenyl]sulfone, bis[4-(3-aminophenoxy)phenyl]sulfone, bis[4-(4-aminophenoxy)phenyl]sulfone, bis[3-(3-aminophenoxy)phenyl]methane, bis[3-(4-aminophenoxy)phenyl]methane, bis[4-(3-aminophenoxy)phenyl]methane, bis[4-(4-aminophenoxy)phenyl]methane, 2,2-bis[3-(3-aminophenoxy)phenyl]propane, 2,2-bis[3-(4-aminophenoxy)phenyl]propane, 2,2-bis[4-(3-aminophenoxy)phenyl]propane, 2,2-bis[4-(4-aminophenoxy)phenyl]propane, 2,2-bis[3-(3-aminophenoxy)phenyl]-1,1,1,3,3,3-hexafluoropropane, 2,2-bis[3-(4-aminophenoxy)phenyl]-1,1,1,3,3,3-hexafluoropropane, 2,2-bis[4-(3-aminophenoxy)phenyl]-1,1,1,3,3,3-hexafluoropropane and 2,2-bis[4-(4-aminophenoxy)phenyl]-1,1,1,3,3,3-hexafluoropropane.

These may be used alone or in mixtures of two or more. The diamine used may be appropriately selected according to the properties desired.

Preferred among these are aromatic diamine compounds, with 3,3'-diaminodiphenyl ether, 3,4'-diaminodiphenyl ether, 4,4'-diaminodiphenyl ether, paraphenylenediamine, 1,3-bis(3-aminophenyl)benzene, 1,3-bis(4-aminophenyl)benzene, 1,4-bis(3-aminophenyl)benzene, 1,4-bis(4-aminophenyl)benzene, 1,3-bis(4-aminophenoxy)benzene and 1,4-bis(3-aminophenoxy)benzene being preferred for use. Particularly preferred is at least one type of diamine selected from the group consisting of benzenediamines, diaminodiphenyl ethers and bis(aminophenoxy)phenyl.

From the viewpoint of heat resistance and dimensional stability under high temperature, the polyimide porous membrane which may be used for the present invention is preferably formed from a polyimide obtained by combination of a tetracarboxylic dianhydride and a diamine, having a glass transition temperature of 240°C or higher, or without a distinct transition point at 300°C or higher.

From the viewpoint of heat resistance and dimensional stability under high temperature, the polyimide porous membrane which may be used for the present invention is preferably a polyimide porous membrane comprising one of the following aromatic polyimides.

(i) An aromatic polyimide comprising at least one tetracarboxylic acid unit selected from the group consisting of biphenyltetracarboxylic acid units and pyromellitic acid units, and an aromatic diamine unit,
(ii) an aromatic polyimide comprising a tetracarboxylic acid unit and at least one type of aromatic diamine unit selected from the group consisting of benzenediamine units, diaminodiphenyl ether units and bis(aminophenoxy)phenyl units, and/or
(iii) an aromatic polyimide comprising at least one type of tetracarboxylic acid unit selected from the group consisting of biphenyltetracarboxylic acid units and pyromellitic acid units, and at least one type of aromatic diamine unit selected from the group consisting of benzenediamine units, diaminodiphenyl ether units and bis(aminophenoxy)phenyl units.

The polyimide porous membrane which may be used for the present invention is preferably a polyimide porous membrane with a three-layer structure comprising a surface layer A and a surface layer B each having a plurality of pores, and a macrovoid layer sandwiched between the surface layer A and the surface layer B, the average pore size of the pores present in the surface layer A is preferably 0.01 $\mu$m or more and 15 $\mu$m or less, and the average pore size of the pores present in the surface layer B is preferably 20 $\mu$m or more and 100 $\mu$m or less. The macrovoid layer preferably has a partition wall jointed to the surface layers A and B and a plurality of macrovoids surrounded by the partition wall and the surface layers A and B, and the thickness of the partition wall of the macrovoid layer and the surface layers A and B is preferably 0.01 $\mu$m or more and 20 $\mu$m or less. The pores in the surface layers A and B communicate with the macrovoids, and the total film thickness is preferably 5 $\mu$m or more and 500 $\mu$m or less. Further, the porosity of the polyimide porous membrane is preferably 40% or more and less than 95%. Here, at least one partition wall in the macrovoid layer may have one or more pores connecting neighboring macrovoids with each other and having an average pore size of 0.01 $\mu$m or more and 100 $\mu$m or less, preferably 0.01 $\mu$m or more and 50 $\mu$m or less.

For example, polyimide porous membranes described in WO 2010/038873, JP 2011-219585 A or JP 2011-219586 A may be used for the present invention.

III-2. Polyethersulfone (PES) porous membrane

The polyethersulfone porous membrane which may be used for the present invention contains polyethersulfone and typically consists substantially of polyethersulfone. Polyethersulfone may be synthesized by the method known to a person skilled in the art. For example, it may be produced by a method wherein a dihydric phenol, an alkaline metal compound and a dihalogenodiphenyl compound are subjected to polycondensation reaction in an organic polar solvent, a method wherein an alkaline metal di-salt of a dihydric phenol synthesized in advance is subjected to polycondensation reaction with a dihalogenodiphenyl compound in an organic polar solvent or the like.

Examples of an alkaline metal compound include alkaline metal carbonate, alkaline metal hydroxide, alkaline metal hydride, alkaline metal alkoxide and the like. Particularly, sodium carbonate and potassium carbonate are preferred.

Examples of a dihydric phenol compound include hydroquinone, catechol, resorcin, 4,4'-biphenol, bis (hydroxyphenyl)alkanes (such as 2,2-bis(hydroxyphenyl)propane, and 2,2-bis(hydroxyphenyl)methane), dihydroxydiphenylsulfones, dihydroxydiphenyl ethers, or those mentioned above having at least one hydrogen on the benzene rings thereof substituted with a lower alkyl group such as a methyl group, an ethyl group, or a propyl group, or with a lower alkoxy group such as a methoxy group, or an ethoxy group. As the dihydric phenol compound, two or more of the aforementioned compounds may be mixed and used.

Polyethersulfone may be a commercially available product. Examples of a commercially available product include SUMIKAEXCEL 7600P, SUMIKAEXCEL 5900P (both manufactured by Sumitomo Chemical Company, Limited).

The logarithmic viscosity of the polyethersulfone is preferably 0.5 or more, more preferably 0.55 or more from the viewpoint of favorable formation of a macrovoid of the polyethersulfone porous membrane; and it is preferably 1.0 or less, more preferably 0.9 or less, further preferably 0.8 or less, particularly preferably 0.75 or less from the viewpoint of the easy production of the polyethersulfone porous membrane.

Further, from the viewpoints of heat resistance and dimensional stability under high temperature, it is preferred that the polyethersulfone porous membrane, or polyethersulfone as a raw material thereof has a glass transition temperature of 200°C or higher, or that a distinct glass transition temperature is not observed.

The method for producing a polyethersulfone porous membrane which may be used for the present invention is not particularly limited, and for example, the polyethersulfone porous membrane may be produced by a method including the following steps:

a step of casting a polyethersulfone solution into a film, the polyethersulfone solution containing 0.3% by mass to 60% by mass of polyethersulfone having a logarithmic viscosity of 0.5 to 1.0 and 40% by mass to 99.7% by mass of an organic polar solvent, and immersing the solution in a coagulating solvent containing a poor solvent or a nonsolvent of polyethersulfone as an essential component or bringing the solution into contact with the coagulating solvent to prepare a coagulated film having pores; and
a step of heat-treating the coagulated film having pores obtained in the above-described step to coarsen the pores, thus obtaining a polyethersulfone porous membrane,
wherein the heat treatment includes raising the temperature of the coagulated film having pores to the glass transition temperature of the polyethersulfone or higher, or 240°C or higher.

The polyethersulfone porous membrane which may be used for the present invention is preferably a polyethersulfone porous membrane having a surface layer A, a surface layer B, and a macrovoid layer sandwiched between the surface layers A and B,

wherein the macrovoid layer has a partition wall jointed to the surface layers A and B, and a plurality of macrovoids surrounded by such a partition wall and the surface layers A and B, the macrovoids having the average pore size in the planar direction of the membrane of 10 to 500 $\mu$m,
wherein the partition wall of the macrovoid layer has a thickness of 0.1 $\mu$m to 50 $\mu$m,
wherein each of the surface layers A and B has a thickness of 0.1 $\mu$m to 50 $\mu$m,
wherein one of the surface layers A and B has a plurality of pores having the average pore size of more than 5 $\mu$m and

200 μm or less, while the other has a plurality of pores having the average pore size of 0.01 μm or more and less than 200 μm,

wherein one of the surface layers A and B has a surface aperture ratio of 15% or more while the other has a surface aperture ratio of 10% or more,

wherein the pores of the surface layers A and B communicate with the macrovoids, and

wherein the polyethersulfone porous membrane has a total film thickness of 5 μm to 500 μm and a porosity of 50% to 95%.

In this way, a cryopreservation substrate formed of a porous membrane can be produced. The cryopreservation substrate according to the present invention may be a porous membrane as it is, may be a laminated porous membrane in which a plurality of porous membranes are laminated, or may be a laminate in which the laminated porous membrane is laminated with another layer such as a mesh.

The cryopreservation substrate thus obtained can also be used as a substrate for cell culture in addition to cryopreservation, so that the load on cells to be cryopreserved can be reduced.

[Examples]

The present invention will now be explained in greater detail by examples. It is to be understood, however, that the present invention is not limited to these examples. A person skilled in the art may easily implement modifications and changes to the present invention based on the description in the present specification, and these are also encompassed within the technical scope of the present invention.

[Experiment 1]

<Polyimide porous membranes>

A polyimide porous membrane having an average film thickness of 25 μm and a porosity of 73% was prepared. The polyimide porous membrane had two different surface layers (A surface and B surface) and a macrovoid layer sandwiched between the two surface layers, and the average pore size of the pores present in the A surface was 6 μm, and the average pore size of the pores present in the B surface was 46 μm.

<Modularized polymer porous membrane>

A modularized polymer porous membrane (hereinafter, also referred to as "module") was obtained by alternately laminating the following laminated polyimide porous membrane (A) and mesh (B) in the order of ABABA, housing the laminate in a polyethylene casing having 16 medium outflow inlets of 2 mm × 2 mm on one surface thereof, and then sterilizing the casing with gamma rays.

Laminated polyimide porous membrane (A): A laminated polyimide porous membrane obtained by laminating six polyimide porous membranes of 1.0 × 1.0 cm in the order of A surface/B surface//A surface/B surface//A surface/B surface//B surface/A surface//B surface/A surface//B surface/A surface was used.

Mesh (B): An insert mesh of 1.0 × 1.0 cm (polypropylene/polyethylene) (manufactured by NBC Meshtec Inc., product number: ESP10TC) was used.

5 wt% FBS-Eagle medium: Eagle medium to which fetal bovine serum (hereinafter, also referred to as "FBS") was added so that the FBS concentration was 5 wt%

2 wt% FBS-Eagle medium: Eagle medium to which FBS was added so that the FBS concentration was 2 wt%

10 wt% FBS-10 wt% DMSO-Eagle medium: Eagle medium to which FBS and DMSO were added so that the FBS concentration was 10 wt% and the DMSO concentration was 10 wt%

The evaluation method in this example is as follows.

<Virus titer>

The culture supernatant was centrifuged at 3,000 rpm for 15 minutes at 4°C, and the supernatant was collected and diluted stepwise 10 times with 5 wt% FBS-Eagle medium to obtain a diluent.

Into a 96-well plate in which pig kidney-derived established cell lines ($1.5 \times 10^5$ cells/mL) suspended in 5% FBS-Eagle medium in advance were seeded at 0.15 mL/well, the diluent was inoculated for each 4 wells at 0.05 mL/well, and the established cell lines were cultured at 37°C in the presence of $5\% CO_2$. On Day 7 of culture, 0.05 mL of each of the supernatants in the respective wells was transferred to a new 96 well V-bottom plate, 0.05 mL of a 0.5 vol% guinea pig red blood cell suspension was added to each well, the plate was allowed to stand at 4°C overnight, and the virus titer was calculated by the Behrens-Karber method using the presence or absence of hemagglutination as an index.

<Viral genome base sequence analysis>

The culture supernatant preserved in an ultra-low temperature freezer was thawed, viral nucleic acid was extracted according to the appended operating procedure manual using QIAmp (registered trademark) DNA Mini Kit (manufactured by QIAGEN K.K.), and a PCR reaction solution was prepared using the extracted nucleic acid as a template, as shown in Table 1. As shown in Table 2, primer sequences were used in the combination of No. 1 (SEQ ID NO. 1) and No. 2 (SEQ ID NO. 2), and the combination of No. 3 (SEQ ID NO. 3) and No. 4 (SEQ ID NO. 4).

[Table 1]

| Table 1 PCR reaction solution | |
|---|---|
| Reagent | Amount |
| TaKaRa Ex Taq (registered trademark) (5U/μL) | 0.25 μL |
| 10 × Ex Taq (registered trademark) Buffer (20 mM) | 5 μL |
| dNTP Mixture (2.5 mM each) | 4 μL |
| Template (genomic DNA) | 10 to 100 ng |
| Primer 1 (10 μM) | 2.5 μL |
| Primer 2 (10 μM) | 2.5 μL |
| Distilled water | up to 50μL |

[Table 2]

| No. | Primer name | Sequence (5'-3') | Product size |
|---|---|---|---|
| 1 | PPV_F2131 | CCAACAACACCAACTTTCAC | 738 bp |
| 2 | PPV_R2868 | GTTGCAGACAATTCAGTGCC | |
| 3 | PPV_F3365 | ACACCAGCAGCACCTAGAAG | 1185 bp |
| 4 | PPV_R4549 | CTAGTATAATTTTCTTGGTATAAG | |

PCR reaction was performed with the program of Table 3 using a thermal cycler.
In the following table, the program from 98°C 10 seconds to 72°C 1 minute means that a total of 30 cycles of a set of performing 98°C 10 seconds, 55°C 30 seconds, and 72°C 1 minute in this order were performed.

[Table 3]

| Table 3 PCR program | | |
|---|---|---|
| Temperature | Time | Cycle |
| 94°C | 2 minutes | 1 cycle |
| 98°C | 10 seconds | 30 cycles as a set |
| 55°C | 30 seconds | |
| 72°C | 1 minute | |
| 72°C | 10 minutes | 1 cycle |

The PCR product was subjected to 2% agarose gel electrophoresis, detection of the band of each of the target sizes shown in Table 2 was confirmed, and the PCR product was purified according to the appended operating procedure manual using NucleoSpin (registered trademark) Gel and PCR Clean-up (manufactured by Takara Bio Inc.).
The base sequence was determined from the purified product by direct sequencing, and mutation analysis was performed. The analysis range was set to a range of bases 2131 to 2868 corresponding to "NS1 gene downstream + intergenic sequence + VP1/VP2 gene upstream" and a range of bases 3365 to 4549 corresponding to "VP1/VP2 gene downstream" in "ACCESSION: NC_001718, Porcine parvovirus complete genome".

(Example 1)

Cryopreservation of pig kidney-derived established cell lines infected with porcine parvovirus

(1) Cell culture of pig kidney-derived established cell lines and inoculation with porcine parvovirus

A solution (45 mL) obtained by adding pig kidney-derived established cell lines ($2.0 \times 10^4$ cells/mL) to a 5% FBS-Eagle medium and five modules, which have been immersed in PBS in advance and shaken for 24 hours, were placed in a 50 mL tube, and rotary culture was performed for 24 hours at 37°C in the presence of 5%$CO_2$ while rotating the tube at 5 revolutions/min.

After 24 hours of the rotary culture, the whole amount of the contents of the 50 mL tube was transferred to a 75 cm$^2$ flask for tissue culture, and static culture was further performed for 6 days at 37°C in the presence of 5%$CO_2$, and then the modules were washed with PBS.

The modules washed with PBS were transferred to a 75 cm$^2$ flask for tissue culture containing 20 mL of a porcine parvovirus solution (hereinafter, also referred to as "virus solution") prepared to have a multiplicity of infection of about 0.01, and the flask was allowed to stand at 37°C in the presence of 5%$CO_2$ for 60 minutes.

Thereafter, the modules were removed from the virus solution and transferred to a new 75 cm$^2$ flask for tissue culture to which 30 mL of 2% FBS-Eagle medium was added, and static culture was started at 37°C in the presence of 5% $CO_2$.

On Day 7 from the virus inoculation culture, the culture supernatant was removed by suction, and 30 mL of 2% FBS-Eagle medium was newly added, followed by culture for another 7 days.

In this way, pig kidney-derived established cell lines infected with porcine parvovirus were cultured for 14 days after virus infection. (Cultured for a total of 14 days after virus inoculation).

(2) Cryopreservation

The pig kidney-derived established cell lines infected with porcine parvovirus, obtained in (1), were removed from the 75 cm$^2$ flask for tissue culture without being detached from the module, and then placed in a cryopreservation tube containing 10 wt% FBS-10 wt% DMSO-Eagle medium. Further, the cryopreservation tube was placed in a BICELL freezing container (manufactured by NIHON FREEZER CO., LTD.), and the BICELL freezing container was placed in an ultra-low temperature freezer at a set temperature of -80°C, followed by freezing by slow cooling.

After freezing overnight, the frozen established cell lines were cryopreserved for 1 month in the ultra-low temperature freezer at the set temperature as it was.

Similarly, the pig kidney-derived established cell lines infected with porcine parvovirus were cryopreserved by changing the 1-month cryopreservation period to 3 months, 6 months, 9 months, and 12 months.

In this way, pig kidney-derived established cell lines infected with porcine parvovirus were cryopreserved for five different cryopreservation periods.

(Reference Example 1)

Culture and cryopreservation were performed in the same cryopreservation periods as in Example 1 except that the established cell lines were cryopreserved in liquid nitrogen instead of being cryopreserved in the ultra-low temperature freezer.

(Example 2)

Culture of pig kidney-derived established cell lines infected with porcine parvovirus after cryopreservation (3) Thawing/re-culturing, supernatant collection

After the end of the cryopreservation period, the cryopreservation tube was taken out from the ultra-low temperature freezer or liquid nitrogen, and quickly thawed in a water bath at 37°C.

After thawing, the modules were removed from the cryopreservation tube, washed in PBS, and then transferred to a 75 cm$^2$ flask for tissue culture containing 30 mL of Eagle medium to which 2% FBS was added, and static culture was resumed at 37°C in the presence of 5%$CO_2$.

On Days 3, 7, 10, 14, 21, and 28 of the re-culture period (Days 17, 21, 24, 28, 35, 42 of total culture), 2 mL of each culture supernatant was collected.

All the remaining culture supernatants were removed at the timing after collection of the supernatants once a week over the entire period, and 30 mL of Eagle medium to which 2%FBS was added was newly added to perform culture.

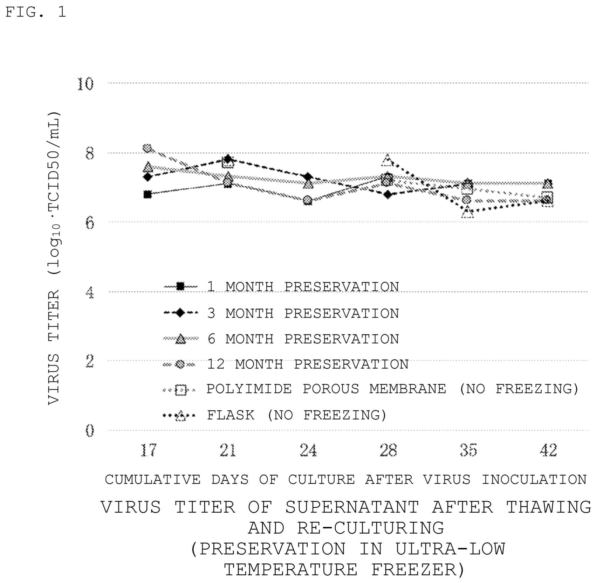

The evaluation results of the pig kidney-derived established cell lines infected with porcine parvovirus using the collected culture supernatants are shown in Tables 4 to 11 and Figs. 1 and 2.

(Reference Example 2)

The pig kidney-derived established cell lines infected with porcine parvovirus were cultured for 42 days in the same manner as in Example 1 except that they were not cryopreserved. On Days 21, 28, 35, and 42 of the culture period, 2 mL of each culture supernatant was collected. The evaluation results of the pig kidney-derived established cell lines infected with porcine parvovirus using the collected supernatants are shown in Tables 4 to 11 and Figs. 1 and 2.

(Reference Example 3)

Pig kidney-derived established cell lines infected with porcine parvovirus were cultured using a flask.

(1) Initial inoculation

Pig kidney-derived established cell lines (20 mL) adjusted to $2.0 \times 10^5$ cells/mL with 5% FBS-Eagle medium were placed in a 75 cm$^2$ flask for tissue culture, and a porcine parvovirus solution was added so that the multiplicity of infection was 0.01, followed by culturing at 37°C in the presence of 5%$CO_2$.

On the next day, the culture supernatant was removed by suction, and 30 mL of a 2% FBS-Eagle medium was newly added, and this was further cultured at 37°C in the presence of 5%$CO_2$ for 6 days (cultured for a total of 7 days after inoculation).

(2) Virus subinoculation, supernatant collection

The culture supernatant (100 μL) of the pig kidney-derived established cell lines inoculated with porcine parvovirus, which had been cultured for 7 days, was collected and added to new pig kidney-derived established cell lines (20 mL) which had been adjusted to $2.0 \times 10^5$ cells/mL with 5% FBS-Eagle medium in advance, and this was cultured at 37°C in the presence of 5%$CO_2$ for 7 days. Thereafter, the operation of inoculating new pig kidney-derived established cell lines was repeated every 7 days of culture.

Then, 2 mL of each of the culture supernatants was collected, and pig kidney-derived established cell lines infected with porcine parvovirus were evaluated using the supernatants. The evaluation results are shown in Tables 4 to 11 and Figs. 1 and 2.

[Table 4]

| Table 4 Virus titer (log10·TCID50/ml) | | | | | | | | | | |
|---|---|---|---|---|---|---|---|---|---|---|
| Cryopreservation method | | Ultra-low temperature freezer | | | | In liquid nitrogen | | | | No freezing | |
| Freezing substrate, and the like | | Polyimide porous membrane | | | | Polyimide porous membrane | | | | Polyimide porous membrane | Flask |
| Cryopreservation period | | 1 month | 3 months | 6 months | 12 months | 1 month | 3 months | 6 months | 12 months | - | - |
| Cumulative inoculation days after virus inoculation | 17 | 6.8 | 7.3 | 7.6 | 8.1 | 8.3 | 7.6 | 7.8 | 7.3 | - | - |
| | 21 | 7.1 | 7.8 | 7.3 | 7.1 | 8.1 | 8.1 | 8.3 | 8.3 | 7.7 | 7.8 |
| | 24 | 6.6 | 7.3 6.8 | 7.1 | 6.6 | 7.8 | 7.8 | 7.8 | 7.1 | - | - |
| | 28 | 7.3 | | 7.3 | 7.1 | 8.1 | 6.1 | 7.3 | 6.6 | 7.2 | 7.8 |
| | 35 | 7.1 | 7.1 | 7.1 | 6.6 | 8.1 | 6.1 | 7.3 | 6.6 | 7.0 | 6.3 |
| | 42 | 7.1 | 7.1 | 7.1 | 6.6 | 7.8 | 6.3 | 7.1 | 6.1 | 6.7 | 6.6 |

[Table 5]

| Table 5 Site at which viral genome mutations were found | | | | | | | |
|---|---|---|---|---|---|---|---|
| SITE | | 3768 | | | | | |
| Cryopreservation method | | Ultra-low temperature freezer | | | | No freezing | |
| Freezing substrate, and the like | | Polyimide porous membrane | | | | Polyimide porous membrane | Flask |
| Cryopreservation period | | 1 month | 3 months | 6 months | 12 months | - | - |
| Cumulative inoculation days after virus inoculation | 17 | T | T | T | T | - | - |
| | 21 | T | T | T | T | T | T |
| | 24 | T | T | T | T | - | - |
| | 28 | T | T | T | T | T | T |
| | 35 | T | T/G | T/G | T/G | T | T |
| | 42 | T/G | T/G | T/G | T/G | T/G | T |

[Table 6]

| Table 6 Site at which viral genome mutations were found | | | | | | | |
|---|---|---|---|---|---|---|---|
| SITE | | 3769 | | | | | |
| Cryopreservation method | | Ultra-low temperature freezer | | | | No freezing | |
| Freezing substrate, and the like | | Polyimide porous membrane | | | | Polyimide porous membrane | Flask |
| Cryopreservation period | | 1 month | 3 months | 6 months | 12 months | - | - |
| Cumulative inoculation days after virus inoculation | 17 | G | G | G | G | - | - |
| | 21 | G | G | G | G | G | G |
| | 24 | G | G | G | G | - | - |
| | 28 | G | G | G/T | G | G | G |
| | 35 | G/T | G/T | G/T | G/T | G | G |
| | 42 | G/T | G/T | G/T | G/T | G/T | G/T |

[Table 7]

| Table 7 Site at which viral genome mutations were found | | | | | | |
|---|---|---|---|---|---|---|
| SITE | | 3955 | | | | |
| Cryopreservation method | | Ultra-low temperature freezer | | | | No freezing | |
| Freezing substrate, and the like | | Polyimide porous membrane | | | | Polyimide porous membrane | Flask |
| Cryopreservation period | | 1 month | 3 months | 6 months | 12 months | - | - |
| Cumulative inoculation days after virus inoculation | 17 | A/C | A/C | A/C | A/C | - | - |
| | 21 | A/C | A/C | A/C | A/C | A/C | A/C |
| | 24 | A/C | A/C | A/C | A/C | - | - |
| | 28 | A/C | A/C | A/C | A/C | A/C | A/C |
| | 35 | A/C | A/C | A/C | C/A | A/C | A/C |
| | 42 | C/A | A/C | A/C | C/A | A/C | A/C |

[Table 8]

| Table 8 Site at which viral genome mutations were found | | | | | | |
|---|---|---|---|---|---|---|
| SITE | | 3980 | | | | |
| Cryopreservation method | | Ultra-low temperature freezer | | | | No freezing | |
| Freezing substrate, and the like | | Polyimide porous membrane | | | | Polyimide porous membrane | Flask |
| Cryopreservation period | | 1 month | 3 months | 6 months | 12 months | - | - |
| Cumulative inoculation days after virus inoculation | 17 | G/C | G/C | G/C | G/C | - | - |
| | 21 | G/C | G/C | G/C | G/C | C/G | G/C |
| | 24 | G/C | G/C | G/C | G/C | - | - |
| | 28 | C/G | G/C | C/G | C/G | C/G | G/C |
| | 35 | G/C | C/G | C/G | C/G | C/G | G/C |
| | 42 | C/G | C/G | C/G | C/G | C/G | G/C |

[Table 9]

| Table 9 Site at which viral genome mutations were found | | | | | | |
|---|---|---|---|---|---|---|
| SITE | | 3942 | | | | |
| Cryopreservation method | | Ultra-low temperature freezer | | | | No freezing | |
| Freezing substrate, and the like | | Polyimide porous membrane | | | | Polyimide porous membrane | Flask |
| Cryopreservation period | | 1 month | 3 months | 6 months | 12 months | - | - |
| Cumulative inoculation days after virus inoculation | 17 | A/G | A/G | A/G | A/G | - | - |
| | 21 | A/G | A/G | A/G | G/A | A/G | G/A |
| | 24 | A/G | A/G | A/G | A/G | - | - |
| | 28 | A/G | G/A | A/G | A/G | A/G | G/A |
| | 35 | A/G | A/G | A/G | A/G | A/G | G/A |
| | 42 | A/G | A/G | A/G | A/G | A/G | G/A |

[Table 10]

| Table 10 Site at which viral genome mutations were found | | | | | | |
|---|---|---|---|---|---|---|
| SITE | | 3973 | | | | |
| Cryopreservation method | | Ultra-low temperature freezer | | | | No freezing | |
| Freezing substrate, and the like | | Polyimide porous membrane | | | | Polyimide porous membrane | Flask |
| Cryopreservation period | | 1 month | 3 months | 6 months | 12 months | - | - |
| Cumulative inoculation days after virus inoculation | 17 | A | A | A | A | - | - |
| | 21 | A | A | A | A | A | A |
| | 24 | A | A | A | A | - | - |
| | 28 | A | A | A | A | A | A |
| | 35 | A | A | A | A | A | A/T |
| | 42 | A | A | A | A | A | T/A |

[Table 11]

| Table 11 Site at which viral genome mutations were found | | | | | | | |
| --- | --- | --- | --- | --- | --- | --- | --- |
| SITE | | 4115 | | | | | |
| Cryopreservation method | | Ultra-low temperature freezer | | | | No freezing | |
| Freezing substrate, and the like | | Polyimide porous membrane | | | | Polyimide porous membrane | Flask |
| Cryopreservation period | | 1 month | 3 months | 6 months | 12 months | - | - |
| Cumulative inoculation days after virus inoculation | 17 | T/C | T/C | T/C | T/C | - | - |
| | 21 | T/C | T/C | T/C | T/C | T/C | T/C |
| | 24 | T/C | T/C | T/C | T/C | - | - |
| | 28 | T/C | T/C | T/C | T/C | T | T/C |
| | 35 | T/C | T/C | T/C | T/C | T | T/C |
| | 42 | T/C | T/C | T | T/C | T | T/C |

From Table 4 and Figs. 1 and 2, it was found that the cells cryopreserved for 12 months in the ultra-low temperature freezer had no change in virus titer for 4 weeks, and the function of the cells was maintained, compared to the cells cryopreserved with liquid nitrogen and the cells not subjected to cryopreservation.

Further, from Tables 5 to 11, the cells cryopreserved in the ultra-low temperature freezer for 12 months had no significant difference in the mutation of the viral genome for 4 weeks, compared to the cells cryopreserved with liquid nitrogen and the cells not subjected to cryopreservation.

[Experiment 2]

As the 6-well insert used in this example, the following was used.

An existing membrane was detached from a cell culture insert for 6 wells (ThinCert (registered trademark) manufactured by Greiner Bio-One International GmbH, for 6 wells, product number 657641), and a polyimide porous membrane was attached to the cell culture insert to prepare an insert. The "polyimide porous membrane" used refers to a polyimide porous membrane having a total film thickness of 25 $\mu$m and a porosity of 73%. The polyimide porous membrane had two different surface layers (A surface and B surface) and a macrovoid layer sandwiched between the two surface layers. The average pore size of the pores present in the A surface was 6 $\mu$m, and the average pore size of the pores present in the B surface was 30 $\mu$m.

In this example, the following was used as the 24-well insert.

An existing membrane was detached from a cell culture insert for 24 wells (ThinCert (registered trademark) manufactured by Greiner Bio-One International GmbH, for 24 wells, product number 662640), and a polyimide porous membrane was attached to the cell culture insert to prepare an insert. The "polyimide porous membrane" used refers to a polyimide porous membrane having a total film thickness of 25 $\mu$m and a porosity of 73%. The polyimide porous membrane had two different surface layers (A surface and B surface) and a macrovoid layer sandwiched between the two surface layers. The average pore size of the pores present in the A surface was 6 $\mu$m, and the average pore size of the pores present in the B surface was 30 $\mu$m.

<Culture and cryopreservation of human fibroblast cell lines>

<Experimental materials and experimental methods>

(1) Expansion of human fibroblasts

To Falcon (registered trademark) Dish (trademark, no treatment, Corning Incorporated), 12 mL of FGM (trademark)-2 Fibroblast Growth Medium-2 BulletKit (trademark, product number: CC-3132, Lonza Group AG) was added, and human fibroblasts were seeded thereon so that the seeding density was 5,000 cells/cm$^2$, and static culture was performed at 37°C in the presence of 5%CO$_2$ for 7 days.

(2) Culture of human fibroblasts using 6-well cell culture insert (Comparative Example 1)

To a 6-well plate (Tissue Culture Plate, product number: 353046, FALCON) was added 2.5 mL of medium per well. A 6-well cell culture insert was placed in each of the wells of the 6-well plate, and this was wetted in an incubator at 37°C in the presence of 5%$CO_2$. To the inside of the wet insert taken out from the incubator was added 2 mL of medium per well. A cell suspension obtained by detaching the human fibroblasts expanded by the method (1) from Falcon (registered trademark) Dish (trademark, no treatment, Corning Incorporated) was seeded on the inside of the insert so that the cell density was 20,000 cells/cm$^2$, and static culture was performed at 37°C in the presence of 5%$CO_2$ for 118 days. The medium was replaced twice a week, and at that time, 0.5 to 1 mL of the medium was collected to obtain a culture supernatant. On Day 9 and Day 118 of culture, the cells were cryopreserved without detaching the cells from the insert.

(3) Culture of human fibroblasts using 24-well cell culture insert (Comparative Example 2)

To a 24-well plate (Tissue Culture Plate, product number: 353047, FALCON) was added 0.5 mL of medium per well. A 24-well cell culture insert was placed in each of the wells of the plate, and this was wetted in an incubator at 37°C in the presence of 5%$CO_2$. To the inside of the wet insert taken out from the incubator was added 0.5 mL of medium per well. A cell suspension obtained by detaching the human fibroblasts expanded by the method (1) from Falcon (registered trademark) Dish (trademark, no treatment, Corning Incorporated) was seeded on the inside of the insert so that the cell density was 20,000 cells/cm$^2$, and static culture was performed at 37°C in the presence of 5%$CO_2$ for 118 days. The medium was replaced twice a week, and at that time, 0.5 to 1 mL of the medium was collected to obtain a culture supernatant. On Day 9 and Day 118 of culture, the cells were cryopreserved without detaching the cells from the insert.

(4) Freezing of human fibroblasts using 6-well cell culture insert

To a 6-well plate (Tissue Culture Plate, product number: 353046, FALCON) was added 2.5 mL of a cell freezing reagent (CELLBANKER1plus, product number: 11912, NIPPON ZENYAKU KOGYO CO., LTD.) per well. The medium on the inside and the outside of the insert, which had been statically cultured for 9 days and 118 days by the method (2), was each collected and removed, and the insert was then placed in each of the wells of a 6-well plate to which a cell freezing reagent had been added in advance. To the inside of the insert was added 2 mL of a freezing reagent per well. The periphery of the 6-well plate was wrapped with Parafilm (product number: PM-996, Bemis Manufacturing Company), then the plate was placed in a zipper bag (bag with zipper, thickness 0.04 mm, product number: 4J, MonotaRO Co., Ltd.), and the zipper bag was placed in an ultra-low temperature freezer (MY BIO, product number: VT-78HC, NIHON FREEZER CO., LTD.) at a set temperature of - 80°C and cryopreserved for about 9 months and 5 months.

(5) Freezing of human fibroblasts using 24-well cell culture insert

To a 24-well plate (Tissue Culture Plate, product number: 353047, FALCON) was added 0.5 mL of a cell freezing reagent per well. The medium on the inside and the outside of the insert, which had been statically cultured for 9 days and 118 days by the method (3), were each collected and removed, and the insert was then placed in each of the wells of a 24-well plate to which a cell freezing reagent had been added in advance. To the inside of the insert was added 0.5 mL of a freezing reagent per well. The periphery of the 24-well plate was wrapped with Parafilm, then the plate was placed in a zipper bag (bag with zipper, thickness 0.04 mm, product number: 4J, MonotaRO Co., Ltd.), and the zipper bag was placed in an ultra-low temperature freezer at a set temperature of -80°C and cryopreserved for about 9 months and 5 months.

<Thawing and re-culturing of human fibroblast cell lines>

(6) Thawing and re-culturing of human fibroblasts using 6-well cell culture insert (Example 1)

To a 6-well plate (Tissue Culture Plate, product number: 353046, FALCON) was added 2.5 mL of PBS (D-PBS (-), product number: 045-29795, FUJIFILM Wako Pure Chemical Corporation) per well. After the end of the cryopreservation period, the 6-well plate was taken out from the ultra-low temperature freezer (-80°C), and quickly thawed in a water bath at 37°C. After taking out the 6-well plate from the water bath, the insert was removed from the dissolved freezing solution and placed in each of wells of a 6-well plate to which PBS was added in advance. To the inside of the insert, 2 mL of PBS was added. After removing PBS, 4.5 mL of PBS was added.

To a 6-well plate (Tissue Culture Plate, product number: 353046, FALCON) was added 2.5 mL of medium per well. The insert immersed in PBS for washing was taken out and placed in a 6-well plate to which medium was added. Then, 2 mL of medium was added thereto. The static culture was resumed at 37°C in the presence of 5%$CO_2$. The medium was replaced twice a week, and at that time, 0.5 to 1 mL of the medium was collected to obtain a culture supernatant.

(7) Thawing and re-culturing of human fibroblasts using 24-well cell culture insert (Example 2)

To a 24-well plate (Tissue Culture Plate, product number: 353047, FALCON) was added 0.5 mL of PBS per well. After the end of the cryopreservation period, the well plate was taken out from the ultra-low temperature freezer (-80°C), and quickly thawed in a water bath at 37°C. After taking out the 24-well plate from the water bath, the insert was removed from the dissolved freezing solution and placed in each of wells of a 24-well plate to which PBS was added in advance. To the inside of the insert, 0.5 mL of PBS was added. After removing PBS, 1 mL of PBS was added.

To a 24-well plate (Tissue Culture Plate, product number: 353047, FALCON) was added 0.5 mL of medium per well. The insert immersed in PBS for washing was taken out and placed in a 6-well plate to which medium was added. To the inside of the insert, 0.5 mL of medium was added. The static culture was resumed at 37°C in the presence of 5%$CO_2$. The medium was replaced twice a week, and at that time, 0.5 to 1 mL of the medium was collected to obtain a culture supernatant.

<Evaluation of culture supernatant>

(11) Measurement of cell product

The concentration of fibronectin contained in each of the culture supernatants collected in Example 1, Example 2, Comparative Example 1, and Comparative Example 2 was measured using an ELISA fibronectin measurement kit (Fibronectin EIAKit, product number: MK115, manufactured by Takara Bio Inc.). The results are shown in Table 12.

[Table 12]

| Table 12 Fibronectin concentration in culture supernatant (ng/mL) | | | | | | | |
|---|---|---|---|---|---|---|---|
| | | 6-well cell culture insert | | | 24-well cell culture insert | | |
| | | Before freezing | Freezing period | | Before freezing | Freezing period | |
| | | | 5 months | 9 months | | 5 months | 9 months |
| Days of culture (days) | 6 | 4,352 | - | - | 2,301 | - | - |
| | 15 | 3,331 | 6,196 | 4,026 | 2,299 | 5,071 | 2,223 |
| | 22 | 4,121 | 11,389 | 7,148 | 2,529 | 9,341 | 4,566 |
| | 40 | 6, 92 6 | - | - | 5,100 | - | - |
| | 116 | 8,147 | - | - | 5, 637 | - | - |

From Table 12, it was found that the cells cryopreserved for 5 months or 9 months in the ultra-low temperature freezer maintained the fibronectin concentration in the culture supernatant, and maintained the fibronectin production function, compared to the cells before freezing.

[Industrial Applicability]

According to the present invention, since cells can be cryopreserved for a long period of time without changing a substrate for cryopreservation, the operation of freezing and thawing the cells is simplified, and the function of the cells can be maintained for a long period of time without using liquid nitrogen. This can significantly reduce the cost of long-term preservation and transportation of frozen cells. In particular, the present invention can greatly contribute to the field of biology including regenerative medicine.

[Reference Signs List]

1 Cell culture insert

2 Cylindrical container having arm structure on upper surface of cylindrical container

3 Porous membrane disposed on bottom surface of cylindrical container

4 Well

## Claims

1. A method for cryopreserving cells, comprising cryopreserving cells for 30 days or more in a state in which the cells are seeded on a porous membrane without using liquid nitrogen.

2. The method according to claim 1, wherein the cells are animal cells.

3. The method according to claim 1, wherein the porous membrane is a polymer porous membrane.

4. The method according to claim 3, wherein an average pore size of a B surface of the polymer porous membrane is larger than an average pore size of an A surface of the polymer porous membrane.

5. The method according to claim 4, wherein the B surface has an average pore size of 10 $\mu$m or more.

6. The method according to claim 4, wherein the A surface has an average pore size of 0.01 $\mu$m or more.

7. The method according to claim 1, wherein the porous membrane has an average pore size of 0.01 $\mu$m or more and 500 $\mu$m or less.

8. The method according to claim 3, wherein the polymer porous membrane is a polyimide porous membrane or a polyethersulfone porous membrane.

9. The method according to claim 3, wherein the porous membrane is a porous membrane provided in a cell culture insert.

10. The method according to claim 2, wherein the animal cells are virus-producing cells or hybridomas.

11. The method according to claim 2, wherein the animal cells are mesenchymal stem cells.

12. The method according to any one of claims 1 to 11, wherein a temperature for cryopreservation is -150°C or higher.

13. A cell culture method comprising the steps of:

    (1) step: seeding cells on a porous membrane;
    (2) step: cryopreserving the cells seeded on the porous membrane for 30 days or more without using liquid nitrogen;
    (3) step: thawing the cells seeded on the porous membrane; and
    (4) step: culturing the thawed cells.

14. The method according to claim 13, wherein the porous membrane is a porous membrane provided in a cell culture insert.

15. A cryopreservation substrate formed of a porous membrane for use in cryopreserving seeded cells for 30 days or more.

16. The cryopreservation substrate according to claim 15, wherein the porous membrane is a porous membrane provided in a cell culture insert.

FIG. 1

VIRUS TITER OF SUPERNATANT AFTER THAWING
AND RE-CULTURING
(PRESERVATION IN ULTRA-LOW
TEMPERATURE FREEZER)

FIG. 2

VIRUS TITER OF SUPERNATANT AFTER THAWING
AND RE-CULTURING
(PRESERVATION IN LIQUID NITROGEN)

FIG. 3

## INTERNATIONAL SEARCH REPORT

| | |
|---|---|
| International application No. | |
| | **PCT/JP2024/021655** |

**A. CLASSIFICATION OF SUBJECT MATTER**

*C12N 5/02*(2006.01)i; *C12M 1/00*(2006.01)i; *C12M 3/00*(2006.01)i; *C12N 5/071*(2010.01)i
FI: C12N5/02; C12N5/071; C12M1/00 A; C12M3/00 A

According to International Patent Classification (IPC) or to both national classification and IPC

**B. FIELDS SEARCHED**

Minimum documentation searched (classification system followed by classification symbols)

C12N5/02; C12M1/00; C12M3/00; C12N5/071

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2020-505033 A (STRATATECH CORPORATION) 20 February 2020 (2020-02-20) claims 11, 16, 23, paragraphs [0024], [0041], [0042], [0048], examples 2, 3 | 1-3, 7, 9, 12-16 |
| X | WO 2016/121767 A1 (UBE INDUSTRIES, LTD.) 04 August 2016 (2016-08-04) claims 23-27, paragraphs [0017], [0046], [0048], [0070], [0088], [0100]-[0102], [0104], example 8, fig. 2 | 1-16 |
| Y | | 8, 10, 12 |
| Y | WO 2021/029409 A1 (UBE INDUSTRIES, LTD.) 18 February 2021 (2021-02-18) claim 1, paragraphs [0015], [0018], [0021], examples | 8, 10, 12 |
| X | JP 2004-057031 A (SANYO ELECTRIC CO., LTD.) 26 February 2004 (2004-02-26) claims 1, 2, paragraphs [0005], [0015], [0022], [0029] | 1-3, 12, 13, 15 |

☐ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **26 August 2024** | **03 September 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

**INTERNATIONAL SEARCH REPORT**

International application No.

**PCT/JP2024/021655**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/JP2024/021655**

| Patent document cited in search report | | | Publication date (day/month/year) | Patent family member(s) | | | Publication date (day/month/year) |
|---|---|---|---|---|---|---|---|
| JP | 2020-505033 | A | 20 February 2020 | US | 2020/0128815 | A1 | |
| | | | | claims 11, 16, paragraphs [0030], [0047], [0048], [0054], examples 2, 3 | | | |
| | | | | WO | 2018/140755 | A1 | |
| | | | | EP | 3573458 | A1 | |
| | | | | CN | 110461149 | A | |
| WO | 2016/121767 | A1 | 04 August 2016 | US | 2017/0369838 | A1 | |
| | | | | claims 23-27, paragraphs [0085], [0133], [0135], [0165], [0187], [0210]-[0213], [0215], example 8, fig. 2 | | | |
| | | | | EP | 3252145 | A1 | |
| | | | | CN | 107208046 | A | |
| | | | | KR | 10-2017-0093250 | A | |
| WO | 2021/029409 | A1 | 18 February 2021 | US | 2022/0290083 | A1 | |
| | | | | claim 1, paragraphs [0028], [0047], [0058], examples | | | |
| | | | | EP | 4012016 | A1 | |
| | | | | CN | 114207109 | A | |
| | | | | KR | 10-2022-0042371 | A | |
| JP | 2004-057031 | A | 26 February 2004 | (Family: none) | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H6209767 A
- WO 2014196549 A
- WO 2016121767 A
- JP 2020171317 A
- JP 2020039326 A
- WO 2009123349 A
- JP 2009057041 W
- WO 2018021368 A
- WO 2010038873 A
- JP 2011219585 A
- JP 2011219586 A

**Non-patent literature cited in the description**

- *Cell*, 2006, vol. 126, 677-689
- *Scientific Reports*, 2016, vol. 6, 1-13